# EUROPEAN PATENT SPECIFICATION

(11) **EP 1 671 620 B1**
(45) Date of publication and mention of the grant of the patent: **16.02.2011**
(21) Application number: 05027598.1
(22) Date of filing: 16.12.2005
(51) Int. Cl.: A61K 8/36, A61Q 1/10

(54) **Makeup composition**
Make-up Zubereitung
Composition de maquillage

(30) Priority: 17.12.2004 JP 2004366417; 17.12.2004 JP 2004366418
(43) Date of publication of application: 21.06.2006
(73) Proprietor: KAO CORPORATION, Chuo-ku, Tokyo (JP)
(72) Inventor: Nakamura, Akane, Tokyo (JP)
(74) Representative: HOFFMANN EITLE

(56) References cited:
- EP-A- 0 898 953
- EP-A- 0 974 338
- US-A1- 2003 143 168
- US-A1- 2004 091 447

## Description

### FIELD OF THE INVENTION

The present invention relates to makeup compositions, particularly, to makeup compositions for conditioning eyelashes or eyebrows into desired shapes to make eyes appear attractive.

### BACKGROUND OF THE INVENTION

Cosmetics are used by men and women generally to enhance their physical beauty. Particularly, cosmetics such as mascara are used by women to condition the shape of eyelashes and eyebrows by coating them individually with mascara, thereby enhancing the attractiveness of eyes. So, cosmetics such as mascara are desired to create long-lasting cosmetic effects.

Such cosmetics are generally formulated from base materials such as solid fats, film-forming agents and pigments. To provide a good feeling upon use and functional effects, the addition of other ingredients with different properties have been examined. For example, to enhance the cosmetic effects such as the curling-up effect and volumizing effect by improving the adhesion of mascara to eyelashes, the addition of a fatty acid such as palmitic acid and stearic acid has been attempted. To make the cosmetic effects last long, it has been attempted to keep eyelashes properly curled for a long period of time by adding a film-forming agent.

To provide mascaras having good cosmetic effects and a good feeling upon use, fatty acid esters and fatty acid soaps each having a C₁₂-C₂₂ alkyl chain have been widely used. For example, JP 2000-119140A discloses an eyelash cosmetic composition containing in combination a specific sucrose fatty acid ester and a fatty acid soap. JP 2003-128522A discloses a cosmetic composition for applying to keratinous fibers, which contains a polyglycerol fatty acid ester and a fatty acid soap. The proposed cosmetic compositions are taught to be excellent in the feeling upon use and the ease of repeated application, form a uniform makeup upon application, and volumize eyelashes.

JP2004-10496A discloses an emulsion-type eyelash cosmetic composition containing a solid oil, a salt of a higher fatty acid and water, in which the feeling upon use and the appearance of makeup obtained are improved and the deterioration over time of the feeling upon use is prevented by the combined use of a higher alcohol, a cyclic polysiloxane and an acrylic alkylamide-acrylic ester copolymer. US-A-2004/091447 and EP-A-898953 disclose oil-in-water make up compositions comprizing fatty acids or their salts, resin emulsions and water.

### SUMMARY OF THE INVENTION

The present invention provides an oil-in-water makeup composition containing (A) from 1 to 40% by weight of a component selected from the group consisting of a carboxylic acid having from 23 to 100 carbon atoms, a salt form thereof, and mixtures thereof, (B) from 1 to 30% by weight on a solid basis of a resin emulsion, (E) from 0.1 to 20 % by weight of a component selected from the groups consisting of a carboxylic acid having from 10 to 22 carbon atoms, a salt form thereof, and (C) water in an amount such that the contents of the ingredients in the makeup composition add up to 100 % by weight.

### DETAILED DESCRIPTION OF THE INVENTION

When an eyelash cosmetic composition contains a fatty acid having a C₁₂-C₂₂ alkyl chain described in the prior art, its coating film disintegrates or dissolves into water or skin oil when coming into contact with a tear or skin oil, or when exposed to friction or force due to blinking, thereby making it difficult to keep the cosmetic effective over a long period of time. If a film-forming agent is added in an increased amount to attain a long-lasting cosmetic effect, the resulting cosmetic composition causes eyelashes to bunch together rather than to form individually differentiated eyelashes when applied to eyelashes, failing to attain well-shaped eyelashes.

The present invention relates to makeup compositions which cause a good curling-up effect and volumizing effect on eyelashes, etc., provide well-shaped eyelashes without causing bunching of the eyelashes, and keep its cosmetic effects for a long period of time.

The makeup composition of the present invention contains (A) a component selected from the group consisting of a carboxylic acid having from 23 to 100 carbon atoms, a salt form thereof, and mixtures thereof. Preferred representatives may be carboxylic acids having the following general formula 1:

RₙCOOH (1)

wherein Rₙ is a straight- or branched-chain, saturated or unsaturated hydrocarbon group having from 22 to 99 carbon atoms in total which may be substituted by a hydroxyl group, provided that a part of the carboxylic acid may be in the form of a salt. When the carbon number of Rₙ is 22 or more, the makeup lasts long, while the carbon number of 99 or less is advantageous with respect to the melting point and viscosity of the makeup compositions. To enhance the above features, the carbon number of Rₙ is preferably 23 or more, and the upper limit thereof is preferably 70 and more preferably 60.

Rₙ is preferably a straight- or branched-chain, saturated hydrocarbon group. To attain a good and waterproof makeup, straight-chain saturated hydrocarbon groups, particularly those having a carboxyl-terminated polyethylene chain are preferred.

The component A, as noted above, is a component selected from the group consisting of a carboxylic acid having from 23 to 100 carbon atoms, a salt form thereof, and mixtures thereof. To improve the feeling upon use, the degree of neutralization of the carboxylic acid is preferably from 0 to 90 mol %, more preferably from 0.1 to 50 mol %, and still more preferably from 0.1 to 30 mol %.

Examples of the counter ions for forming the salt of a carboxylic acid having from 23 to 100 carbon atoms include, but are not limited to, ions derived from alkali metals such as sodium and potassium; organic amines such as monoethanolamine, diethanolamine, triethanolamine, aminomethylpropanol, aminomethylpropanediol, tris(hydroxymethyl)aminomethane and morpholine; and a basic amino acid such as L-arginine, with the organic amines being preferred and aminomethylpropanol and aminomethylpropanediol being more preferred. These substances may be added to the makeup composition as a neutralizing agent. The amount of use may be suitably determined according to the content of the carboxylic acid in the makeup composition and the desired degree of neutralization thereof, and is preferably from 0.01 to 10% by weight, more preferably from 0.1 to 5% by weight, each based on the makeup composition. If the makeup composition contains an additional carboxylic acid other than the component A, for example the component (E) which will be described below, the neutralizing agent also neutralize such an additional carboxylic acid.

Products containing the carboxylic acids having from 23 to 100 carbon atoms are commercially available under the trade names "Performacid 350ACID" (New Phase Technologies, Ltd.), "Syncrowax AW1-C" (Croda Inc.), etc. for products containing straight-chain carboxylic acids, and "Yofco Lanolin Fatty Acid HH" (Nippon Fine Chemical Co., Ltd.) for products containing straight-chain carboxylic acids and branched-chain carboxylic acids.

The content of the component A is preferably from 1 to 40% by weight, more preferably from 1.5 to 20% by weight, and even more preferably from 2.5 to 10% by weight, each based on the total amount of the makeup composition, because a good feeling upon use and a long-lasting makeup are obtained.

The resin emulsion B used in the present invention is an emulsion obtained by dispersing a polymer in a dispersion medium mainly composed of water. Particularly preferred is an emulsion that is prepared by the polymerization in water of a monomer having a polymerizable double bond in the presence of a plasticizer or a film-forming aid. The glass transition temperature (Tg) of the polymer in the resin emulsion B is preferably from -30 to 65°C and more preferably from -20 to 45°C because a soft makeup film can be obtained.

The monomer having a polymerizable double bond is either hydrophilic or hydrophobic. Examples of the hydrophilic monomers include ethylenically unsaturated carboxylic acids such as acrylic acid, methacrylic acid, itaconic acid, maleic acid, fumaric acid and crotonic acid; ethylenic monomers having a hydroxyl group such as hydroxyethyl (meth)acrylate, glycidyl (meth)acrylate, ethylene glycol di(meth)acrylate, polyethylene glycol mono(meth)acrylate; ethylenic amides such as (meth)acrylamide, N-methylol(meth)acrylamide and N-diacetoneacrylamide; ethylenic amines such as aminoethyl (meth)acrylate, N,N-dimethylaminoethyl (meth)acrylate, N,N-diethyleaminoethyl (meth)acrylate and N,N,N-trimethylaminoethyl (meth) acrylate; and salts of the ethylenic amines.

Examples of the hydrophobic monomers include aromatic mono- or divinyl compounds such as styrene, α-methylstyrene, chlorostyrene, alkylstyrene and divinylbenzene; (meth)acrylates such as methyl (meth)acrylate, ethyl (meth)acrylate, propyl (meth)acrylate, butyl (meth)acrylate, 2-ethylhexyl (meth)acrylate, tert-butyl (meth)acrylate and cyclohexyl (meth)acrylate; vinyl cyanide compounds such as acrylonitrile and methacrylonitrile; vinyl esters such as vinyl acetate; vinyl halides such as vinyl chloride and vinylidene chloride; fluorine-containing monomers such as trifluoroethyl methacrylate, 2,2,3,3-tetrafluoropropyl methacrylate, 2,2,3,3,4,4-hexafluorobutyl methacrylate, perfluorooctyl methacrylate and perfluorooctyl acrylate; and silicone macromonomers. The term "(meth)acryl" means acryl or methacryl.

These monomers may be used alone or in combination of two or more. If used in combination, the combination is preferably composed of from 0 to 30% by weight of the hydrophilic monomer and from 70 to 100% by weight of the hydrophobic monomer, and more preferably composed of from 0 to 15% by weight of the hydrophilic monomer and from 85 to 100% by weight of the hydrophobic monomer.

As the resin emulsion B, preferred are alkyl (meth)acrylate polymer emulsions, alkyl (meth)acrylate copolymer emulsions, (meth)acrylic acid-alkyl (meth)acrylate copolymer emulsions, alkyl (meth)acrylate-styrene copolymer emulsions, vinyl acetate polymer emulsions, vinyl acetate-containing copolymer emulsions, vinylpyrrolidone-styrene copolymer emulsions, and silicone-containing polymer emulsions. These resin emulsions can be commercially available, for example, under the trade names "Yodosol GH34" (Nippon NSC, Ltd.; solid content = 45 to 47% by weight; Tg = -16°C), "Yodosol GH810" (Nippon NSC, Ltd.; solid content = 45 to 47% by weight; Tg = 20°C), "Daitosol 5000AD" (Daito Kasei Kogyo Co., Ltd.; solid content = 48 to 52% by weight; Tg = -14°C), "Jurymer ET-416" (alkyl acrylate polymer emulsion; Nihon Junyaku Co., Ltd.; solid content = 56 to 58% by weight; Tg = 0°C), and "Jurymer ET-410" (alkyl acrylate copolymer emulsion; Nihon Junyaku Co., Ltd.; solid content = 28.5 to 31.5% by weight; Tg = 44°C).

Examples of the compounds used as the plasticizer or the film-forming aid in the preparation of the resin emulsion B include Cellosolves of Union Carbide such as Cellosolve (ethylene glycol ethyl ether), methyl Cellosolve (ethylene glycol methyl ether) and butyl Cellosolve (ethylene glycol butyl ether); Carbitols of Union Carbide such as Carbitol (diethylene glycol ethyl ether), methyl Carbitol (diethylene glycol methyl ether), butyl Carbitol (diethylene glycol butyl ether) and dibutyl Carbitol (diethylene glycol dibutyl ether); carbonates such as ethylene carbonate and propylene carbonate; acetates such as Cellosolve acetate (ethylene glycol ethyl ether acetate), butyl Cellosolve acetate (ethylene glycol butyl ether acetate), butyl Carbitol acetate (diethylene glycol butyl ether acetate) and sucrose acetate; alcohols such as hexanol, benzyl alcohol and phenethyl alcohol; diols such as hexylene glycol, ethylene glycol and propylene glycol; esters such as phthalic acid diesters, adipic acid diesters, succinic acid diesters, sebacic acid diesters, abietic acid esters, caprylic acid esters, caproic acid esters, acetic acid esters, enanthic acid esters, myristic acid esters and citric acid esters; benzoic acid esters such as sucrose benzoate; and diethylbenzene.

Taking the polymerization stability, storage stability, water resistance and durability of coating film into account, the plasticizer or the film-forming aid is added preferably from 1 to 50 parts by weight and more preferably from 5 to 30 part by weight to 100 parts by weight of the monomer. The plasticizer or the film-forming aid is added either before the initiation of polymerization or during the polymerization.

The polymerization initiators to be used may include organic polymerization initiators, for example, hydroperoxides such as cumene hydroperoxide, diisopropylbenzene hydroperoxide and p-menthane hydroperoxide, peroxides such as benzoyl peroxide and lauroyl peroxide, and azo compounds such as azobisisobutyronitrile; and inorganic polymerization initiators, for example, persulfates such as potassium persulfate, sodium persulfate and ammonium persulfate, although not limited thereto. Also usable are so-called redox polymerization initiators where the polymerization initiator is combined with a reducing agent such as sodium bisulfite, ascorbic acid and its salt.

To stabilize the dispersed state, the polymerization may be carried out in the presence of an anionic, cationic or nonionic surfactant. A chain transfer agent is also usable.

The polymerization for preparing the resin emulsion B may be performed by a known method such as emulsion polymerization, solution polymerization, bulk polymerization, precipitation polymerization and non-emulsion polymerization. The weight average molecular weight of the resultant resin in the emulsion is preferably 200,000 or less, and more preferably from 10,000 to 100,000.

These resin emulsions B may be used alone or in combination of two or more. If used in combination, such a combination may contain two or more polymers having different glass transition temperatures. The resin emulsion B is contained in the makeup compositions in an amount ranging from 1 to 30% by weight on a solid basis. To enhance the film-forming ability and the cleansing properties, its content on a solid basis is preferably from 2 to 20% by weight.

The makeup composition of the present invention is in a form of an oil-in-water emulsion containing a balance of water as the component C. The content range of water is selected so as to allow the contents of the ingredients in the makeup composition to add up to 100% by weight, with a content ranging from 30 to 98% by weight being preferred, because the drying speed of coated films can be properly controlled. To facilitate the control of drying speed, the upper limit of the water content is preferably 70% by weight and more preferably 60% by weight.

In addition to the component A, component B and component C, the makeup composition of the present invention may further contain a wax ester having from 24 to 100 carbon atoms as the component D (also called "wax ester D"). The wax esters referred to herein are esters of higher fatty acids and/or hydroxycarboxylic acids with higher monohydric alcohols, which have a total carbon number of from 24 to 100 and which are solids at 25°C preferably having a melting point of 50°C or higher. The carbon number is preferably from 12 to 50 for both the higher fatty acids and the higher monohydric alcohols. By using the wax ester D, a desired curling effect and volumizing effect are caused on eyelashes, etc. To obtain a desired hardness of the coated film, the total carbon number of the wax esters D is preferably from 30 to 70.

Examples of the wax esters D include waxes of vegetable origin such as carnauba wax, rice wax, jojoba wax (inclusive of extreme hydrogenated jojoba oil) and candelilla wax; and waxes of animal origin such as spermaceti wax, bees wax and ibota wax.

To obtain the curling-up effect and volumizing effect, the component D is preferably contained in an amount of from 1 to 40% by weight based on the total amount of the makeup composition. To enhance the feeling upon use, its content is preferably from 2 to 30% by weight and more preferably from 2.5 to 20% by weight.

The makeup composition of the present invention contains, as a component E, a saturated or unsaturated carboxylic acid having from 10 to 22 carbon atoms, which may partly or completely neutralized to form a salt. Examples thereof include carboxylic acids such as lauric acid, myristic acid, palmitic acid, stearic acid, behenic acid, oleic acid, undecylenic acid, isostearic acid, linoleic acid, linolenic acid, eicosapentaenoic acid, docosahexaenoic acid, coconut oil fatty acid, palm oil fatty acid and tallow fatty acid; and salts of these carboxylic acids. To make the coated film smooth, the content of the component E is preferably from 0.1 to 20% by weight and more preferably from 2 to 15% by weight based on the total amount of the makeup composition.

If the makeup composition contains, in addition to the component A, a carboxylic acid having 22 or less of carbon atoms and/or its salt, a weight ratio of Y/X, wherein X is the weight of all of the carboxylic acids inclusive of their salts and Y is the weight of the carboxylic acid serving as the component A, each contained in the makeup composition, is preferably 0.21 or more, more preferably from 0.25 to 1.0, and still more preferably from 0.5 to 0.9, because the cosmetic effect lasts long and a flexible coated film is obtained.

To stabilize the makeup composition, the weight ratio Z/X, wherein X is the same as defined above and Z is the weight of the component D contained in the makeup composition, is preferably from 2 to 20 and more preferably from 3 to 5.

To regulate the hardness of the coated film, the makeup composition may contain a hydrocarbon oil which is solid at 25°C. Examples thereof include micro crystalline wax, paraffin wax, polyethylene wax, silicone wax and ceresin. The content thereof in the makeup composition is preferably from 0.5 to 20% by weight and more preferably from 0.8 to 10% by weight.

The makeup composition may further contain, if necessary, an emulsifier such as surfactants. To maintain a stable oil-in-water emulsion, usable are cosmetically acceptable surfactants such as nonionic surfactants, anionic surfactants, cationic surfactants and amphoteric surfactants, with the nonionic surfactant being preferred because of their compatibility with other ingredients of the makeup composition. Examples of the nonionic surfactants include glycerol fatty acid ester, polyglycerol fatty acid ester, propylene glycol fatty acid ester, sorbitan fatty acid ester and alkylene glycol adducts thereof, sorbitol fatty acid ester and alkylene glycol adducts thereof, polyalkylene glycol fatty acid ester, sucrose fatty acid ester, polyoxyalkylene alkyl ether, glycerol alkyl ether, polyoxyethylene hydrogenated castor oil, lanolin-alkylene glycol adducts and polyoxyalkylene alkyl ether co-modified silicone, with polyalkylene glycol fatty acid ester, sucrose fatty acid ester, polyoxyalkylene alkyl ether and polyoxyethylene hydrogenated castor oil being preferred. The content of the surfactant in the makeup composition is preferably from 0.5 to 10% by weight and more preferably from 1 to 5% by weight.

The makeup composition may further contain, in addition to the components described above, another cosmetically acceptable component, for example, a powder component. Examples thereof include inorganic powders such as prussian blue, ultramarine blue, red oxide, iron oxide, titanium oxide, zinc oxide, silicon dioxide, carbon black, magnesium silicate, aluminum magnesium silicate, mica, synthetic mica, synthetic sericite, sericite, talc, kaolin, silicon carbide, barium sulfate, bentonite and smectite; brilliant powders such as mica titanium, iron oxide mica and aluminum powder; pigment powders such as organic tar pigments and lake pigments of organic dyestuff; and composite powders such as mica titanium coated with titanium oxide fine particles, mica titanium coated with zinc oxide fine particles and mica titanium coated with barium sulfate fine particles.

These powders can be used after being surface-treated with silicone, higher fatty acid, higher alcohol, fatty acid ester, metallic soap, amino acid, alkyl phosphate, etc., or after being encapsulated into organic or inorganic microcapsules. If the powders are incorporated into the oil phase, it is preferred to subject the surface of the powder to hydrophobic treatment, preferably by silicone treatment or acrylsilicone treatment, because the dispersibility and high-temperature storage stability can be improved.

The powders described above may be used singly or in combination of two or more.

The makeup composition may further contain a higher alcohol, a water-soluble polymer, alcohols, polyhydric alcohols, chemicals, an oil such as hydrocarbon oil silicone oil, a thickener, an ultraviolet absorber, an ultraviolet diffuser, a humectant, a fragrance, an antioxidant, etc.

When the makeup composition is formulated into mascara, fibers may be included to enhance the long lash effect. Any of the natural fibers such as cotton, silk and hemp; regenerated fibers such as rayon; and synthetic fibers such as polyamide, polyester, acrylic resin and polyethylene can be used for such purpose, with fibers of a polyamide such as nylon being preferred because of their toughness. The surface of the fibers may, if necessary, be treated before use, for example, by silica treatment, silicone treatment, fluorine treatment, metallic soap treatment or oil and fat treatment in order to improve the dispersibility in oil. To enhance the adhesion to eyelashes, the fineness of fibers is preferably from 1.0 to 25.0 T (T: weight of fiber per 1000 m), and the fiber length is preferably from 0.1 to 5 mm. To attain a sufficient long lash effect, the content of fibers is preferably from 0.1 to 6% by weight based on the total weight of the makeup composition.

The total content of the optional components such as powders and fibers is suitably selected from the range of from 1 to 50% by weight based on the total weight of the makeup composition.

The makeup composition is prepared by a known method, e.g., by mixing the components mentioned above into a uniform mixture while stirring.

The viscosity of the makeup composition at 25°C is preferably from 100 to 2000 Pa·s and more preferably from 150 to 1000 Pa·s. Within the above range, the adhesion to eyelashes is good, the makeup obtained has a good appearance, and the production thereof is facilitated.

The makeup composition, when applied to eyelashes, is uniformly coated on and along individual eyelashes to cause a good curling-up effect and volumizing effect on eyelashes. In addition, the eyelashes are well shaped without bunching together upon application and the cosmetic effect lasts long.

The makeup composition of the invention is suitable preferably as eyelash makeup, e.g., as mascara, and also suitable as eyebrow makeup, i.e., eyebrow mascara. In addition, the makeup composition may not only be formulated into pigmented products, but also used as a pre-makeup or top coat for making-up eyelashes.

The following examples further describe and demonstrate embodiments of the present invention.

### EXAMPLES 1-4 and COMPARATIVE EXAMPLES 1-2

Each eyelash makeup having a composition shown in Table 1 was prepared. The contents (percent by weight) of the components 1-31 and the results of evaluation are also shown in Table 1.

The fat(s) and carboxylic acid(s) were melted at 90°C or higher under heating, and then added into an aqueous solution kept at 85°C or higher which contained a neutralizing agent, emulsifier, water-soluble polymer, 1,3-butylene glycol, preservative (methylparaben) and colorant (black iron oxide). The mixture was made into a dispersion using a homomixer and then cooled to room temperature while stirring. After cooling, the rest of the components such as the resin emulsion and ethanol were added to prepare each eyelash makeup.

### Evaluation Method

The evaluation was carried out by ten professional panelists. Each panelist applied the eyelash makeup stored in a container to her eyelashes in her own manner, and rated herself the appearance of the makeup obtained according to the following criteria. The result of the evaluation is expressed by the average of ratings given by ten panelists.

### Evaluated properties

(1) Resistance to bunching of eyelashes
(2) Curling-up and volumizing effect
(3) Durability of cosmetic effect determined by the comparison of observations made immediately after application and at three hours after application.

### Evaluation criteria

5: Very good
4: Good
3: Normal
2: Poor
1: Very poor

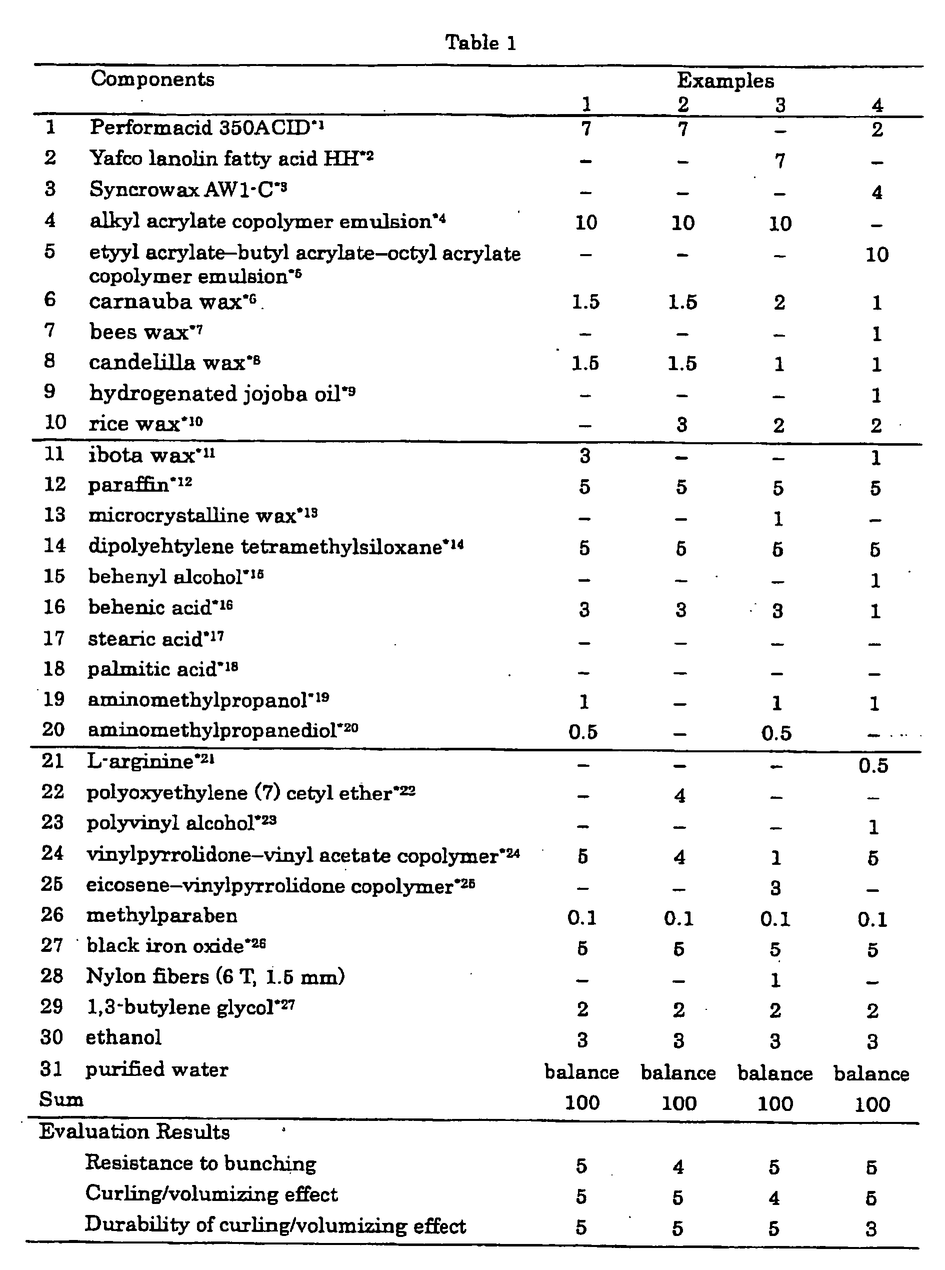

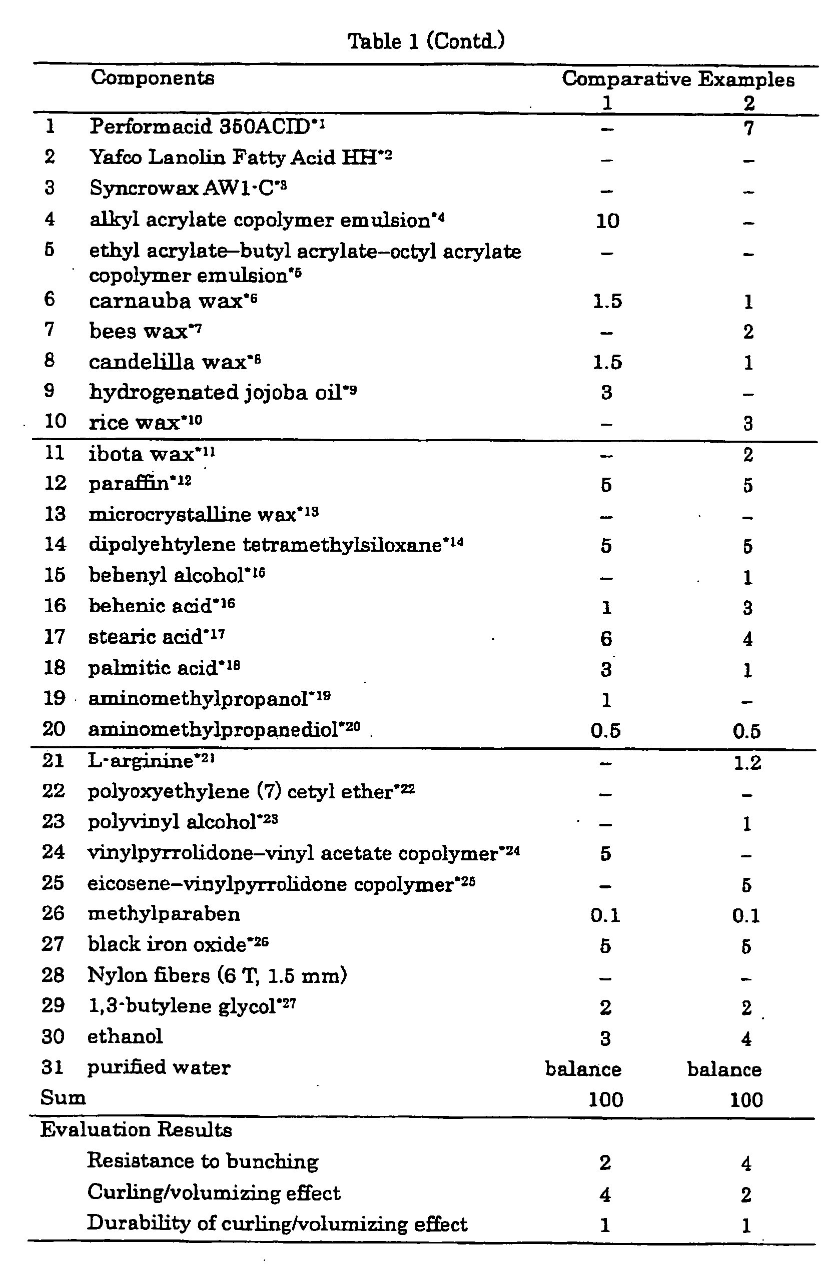

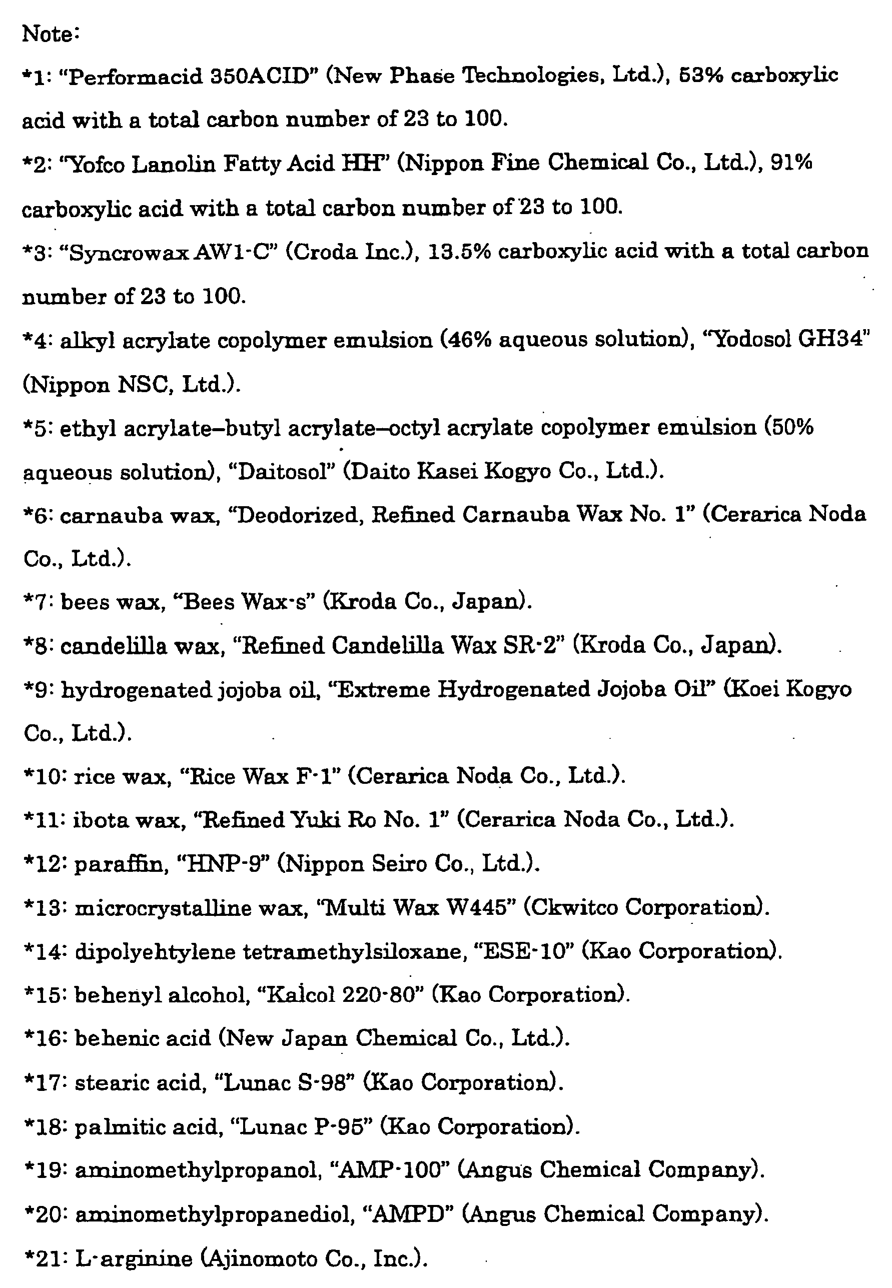

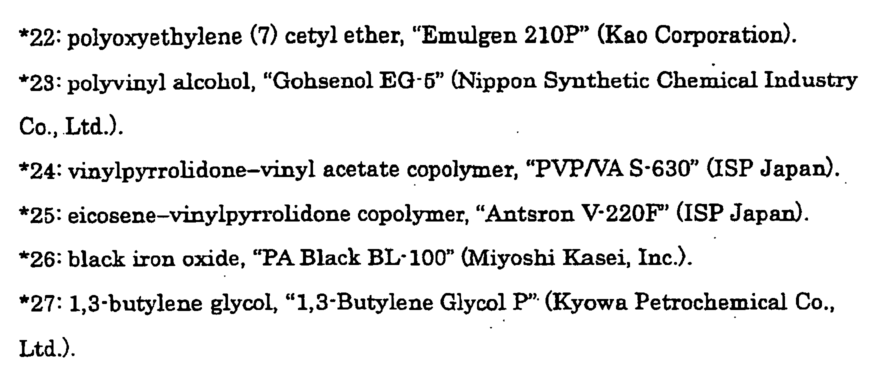

### EXAMPLE 5

A mascara having the following composition was prepared.

| | | % by weight |
|---|---|---|
| (1) | "Performacid 350ACID" (New Phase Technologies, Ltd.) 53% carboxylic acid with a total carbon number of 23 to 100 | 7.0 |
| (2) | Alkyl acrylate copolymer emulsion "Daitosol" (Daito Kasei Kogyo Co., Ltd.) | 10.0 |
| (3) | carnauba wax "Deodorized, Refined Carnauba Wax No. 1" (Cerarica Noda Co., Ltd.) | 1.5 |
| (4) | candelilla wax "Refined Candelilla Wax SR-2" (Kroda Co., Japan) | 1.5 |
| (5) | ibota wax "Refined Yuki Ro No. 1" (Cerarica Noda Co., Ltd.) | 3.0 |
| (6) | paraffin, "HNP-9" (Nippon Seiro Co., Ltd.) | 5.0 |
| (7) | dipolyehtylene tetramethylsiloxane "ESE-10" (Kao Corporation) | 5.0 |
| (8) | behenic acid (New Japan Chemical Co., Ltd.) | 3.0 |
| (9) | aminomethylpropanol "AMP-100" (Angus Chemical Company) | 1.0 |
| (10) | aminomethylpropanediol "AMPD" (Angus Chemical Company) | 0.5 |
| (11) | vinylpyrrolidone-vinyl acetate copolymer "PVP/VA S-630" (ISP Japan) | 5.0 |
| (12) | 1,3-butylene glycol "1,3-Butylene Glycol P" (Kyowa Petrochemical Co., Ltd.) | 2.0 |
| (13) | ethanol | 3.0 |
| (14) | methylparaben | 0.15 |
| (15) | propylparaben | 0.05 |
| (16) | phenoxyethanol | 0.4 |
| (17) | black iron oxide, "PA Black BL-100" (Miyoshi Kasei, Inc.) | 5.0 |
| (18) | purified water | balance |

The mascara thus prepared was evaluated in the same manner as in Example 1, and was found to be sufficient in the resistance to bunching, curling-up effect, volumizing effect and durability of such effects.

### EXAMPLE 6

A mascara having the following composition was prepared.

| | | % by weight |
|---|---|---|
| (1) | "Performacid 350ACID" (New Phase Technologies, Ltd.) 53% carboxylic acid with a total carbon number of 23 to 100 | 5.0 |
| (2) | Alkyl acrylate copolymer emulsion "Datiosol" (Daito Kasei Kogyo Co., Ltd.) | 15.0 |
| (3) | carnauba wax "Deodorized, Refined Carnauba Wax No. 1" (Cerarica Noda Co., Ltd.) | 3.0 |
| (4) | candelilla wax "Refined Candelilla Wax SR-2" (Kroda Co., Japan) | 1.5 |
| (5) | extreme hydrogenated jojoba oil (Koei Kogyo Co., Ltd.) | 4.0 |
| (6) | rice wax, "Rice Wax F-1" (Cerarica Noda Co., Ltd.) | 3.0 |
| (7) | cetyl alcohol "Cetyl Alcohol NX" (Kokyu Alcohol Kogyo Co., Ltd.) | 1.2 |
| (8) | stearyl alcohol | 0.8 |
| | "Stearyl Alcohol NX" (Kokyu Alcohol Kogyo Co., Ltd.) | |
| (9) | behenic acid (New Japan Chemical Co., Ltd.) | 1.0 |
| (10) | stearic acid, "Lunac S-98" (Kao Corporation) | 2.0 |
| (11) | aminomethylpropanol "AMP-100" (Angus Chemical Company) | 1.0 |
| (12) | aminomethylpropanediol "AMPD" (Angus Chemical Company) | 0.5 |
| (13) | vinylpyrrolidone-vinyl acetate copolymer "PVP/VA S-630" (ISP Japan) | 2.0 |
| (14) | 1,3-butylene glycol "1,3-Butylene Glycol P" (Kyowa Petrochemical Co., Ltd.) | 3.0 |
| (15) | methylparaben | 0.15 |
| (16) | propylparaben | 0.05 |
| (17) | phenoxyethanol | 0.4 |
| (18) | black iron oxide, "PA Black BL-100" (Miyoshi Kasei, Inc.) | 5.0 |
| (19) | purified water | balance |

The mascara thus prepared was evaluated in the same manner as in Example 1, and was found that to be sufficient in the resistance to bunching, curling-up effect, volumizing effect and durability of such effects.

### EXAMPLE 7

A mascara having the following composition was prepared. The weight ratio Y/X, wherein X is the weight of total carboxylic acids in the mascara and Y is the weight of a carboxylic acid having from 23 to 100 carbon atoms, was 0.33.

| | | % by weight |
|---|---|---|
| (1) | "Performacid 350ACID" (New Phase Technologies, Ltd.) 53% carboxylic acid with a total carbon number of 23 to 100 | 6.0 |
| (2) | Alkyl acrylate copolymer emulsion (46% aqueous solution) "Yodosol GH34" (Nippon NSC, Ltd.) | 15.0 |
| (3) | carnauba wax "Deodorized, Refined Carnauba Wax No. 1" (Cerarica Noda Co., Ltd.) | 3.5 |
| (4) | candelilla wax "Refined Candelilla Wax SR-2" (Kroda Co., Japan) | 1.5 |
| (5) | rice wax, "Rice Wax F-1" (Cerarica Noda Co., Ltd.) | 3.5 |
| (6) | dipolyehtylene tetramethylsiloxane "ESE-10" (Kao Corporation) | 2.0 |
| (7) | cetyl alcohol "Cetyl Alcohol NX" (Kokyu Alcohol Kogyo Co., Ltd.) | 2.0 |
| (8) | behenic acid (New Japan Chemical Co., Ltd.) | 1.5 |
| (9) | stearic acid, "Lunac S-98" (Kao Corporation) | 2.0 |
| (10) | aminomethylpropanol "AMP-100" (Angus Chemical Company) | 1.0 |
| (11) | vinylpyrrolidone-vinyl acetate copolymer "PVP/VA S-630" (ISP Japan) | 2.0 |
| (12) | 1,3-butylene glycol "1,3-Butylene Glycol P" (Kyowa Petrochemical Co., Ltd.) | 3.0 |
| (13) | ethanol | 1.0 |
| (14) | methylparaben | 0.15 |
| (15) | propylparaben | 0.05 |
| (16) | phenoxyethanol | 0.5 |
| (17) | black iron oxide, "PA Black BL-100" (Miyoshi Kasei, Inc.) | 5.0 |
| (18) | purified water | balance |

The mascara thus prepared was evaluated in the same manner as in Example 1, and was found to be sufficient in the resistance to bunching, curling-up effect, volumizing effect and durability of such effects.

The makeup composition of the invention, when used as a mascara for eyelashes, causes a good and long-lasting curling-up effect and volumizing effect on individual eyelashes.

## Claims

1. An oil-in-water makeup composition comprising (A) from 1 to 40 % by weight of a component selected from the group consisting of a carboxylic acid having from 23 to 100 carbon atoms, a salt form thereof, and mixtures thereof, (B) from 1 to 30% by weight on a solid basis of a resin emulsion, (E) from 0.1 to 20 % by weight of a component selected from the groups consisting of a carboxylic acid having from 10 to 22 carbon atoms, a salt form thereof, and (C) water in an amount such that the contents of the ingredients in the makeup composition add up to 100% by weight.

2. The oil-in-water makeup composition according to Claim 1 further comprising (D) from 1 to 40 % by weight of a wax ester having from 24 to 100 carbon atoms.

3. The oil-in-water makeup composition according to Claim 1 or 2, wherein a weight ratio of Y/X, wherein X is a weight of all of the carboxylic acids inclusive of their salt and Y is a weight of the carboxylic acid as the component A, each contained in the makeup composition, is 0.21 or more.

4. The oil-in-water makeup composition according to any of Claims 1 to 3, wherein the component (A) is a carboxylic acid having a straight or branched chain, saturated hydrocarbon group.

5. The oil-in-water makeup composition according to Claim 4, wherein the component (A) has a carboxyl group at a terminal end of a polyethylene chain.

6. Mascara formulation comprising the oil-in-water makeup composition according to anyone of Claims 1 to 5.

7. Use of the oil-in-water composition of any one of claims 1 to 5 as a makeup composition.

## Patentansprüche

1. Öl-in-Wasser-Makeup-Zusammensetzung, umfassend (A) von 1 bis 40 Gew.-% einer Komponente, ausgewählt aus der Gruppe bestehend aus einer Carbonsäure mit 23 bis 100 Kohlenstoffatomen, eine Salzform davon und Mischungen davon, (B) von 1 bis 30 Gew.-%, bezogen auf Feststoffbasis einer Harzemulsion, (E) von 0,1 bis 20 Gew.-% einer Komponente, ausgewählt aus der Gruppe bestehend aus einer Carbonsäure mit 10 bis 22 Kohlenstoffatomen, einer Salzform davon und (C) Wasser in einer Menge, so dass der Gehalt der Bestandteile in der Makeup-Zusammensetzung sich auf 100 Gew.-% ergänzt.

2. Öl-in-Wasser-Makeup-Zusammensetzung nach Anspruch 1, weiterhin umfassend (D) von 1 bis 40 Gew.-% eines Wachsesters mit 24 bis 100 Kohlenstoffatomen.

3. Öl-in-Wasser-Makeup-Zusammensetzung nach Anspruch 1 oder 2, worin das Gewichtsverhältnis von Y/X 0,21 oder mehr ist, worin X ein Gewicht aller Carbonsäuren, einschließlich ihrer Salze, ist und Y ein Gewicht der Carbonsäure als Komponente (A) ist, jeweils enthalten in der Makeup-Zusammensetzung.

4. Öl-in-Wasser-Makeup-Zusammensetzung nach einem der Ansprüche 1 bis 3, worin die Komponente (A) eine Carbonsäure mit einer geradkettigen oder verzweigten gesättigten Kohlenwasserstoffgruppe ist.

5. Öl-in-Wasser-Makeup-Zusammensetzung nach Anspruch 4, worin die Komponente (A) eine Carboxylgruppe an einem Ende einer Polyethylenkette davon hat.

6. Maskaraformulierung, umfassend die Öl-in-Wasser-Makeup-Zusammensetzung nach einem der Ansprüche 1 bis 5.

7. Verwendung der Öl-in-Wasser-Makeup-Zusammensetzung nach einem der Ansprüche 1 bis 5 als Makeup-Zusammensetzung.

## Revendications

1. Composition de maquillage huile dans l'eau comprenant (A) de 1 à 40% en poids d'un composant choisi parmi le groupe consistant en un acide carboxylique ayant de 23 à 100 atomes de carbone, une forme de sel de celui-ci, et des mélanges de ceux-ci, (B) de 1 à 30% en poids sur la base des solides d'une émulsion de résine, (E) de 0,1 à 20% en poids d'un composant choisi parmi le groupe consistant en un acide carboxylique ayant de 10 à 22 atomes de carbone, une forme de sel de celui-ci, et (C) de l'eau dans une quantité telle que les teneurs des ingrédients dans la composition de maquillage constituent 100% en poids.

2. Composition de maquillage huile dans l'eau selon la revendication 1 comprenant en outre (D) de 1 à 40% en poids d'un ester de cire ayant de 24 à 100 atomes de carbone.

3. Composition de maquillage huile dans l'eau selon la revendication 1 ou 2, dans laquelle un rapport en poids de Y/X, dans lequel X est un poids de la totalité des acides carboxyliques, y compris leur sel, et Y est un poids de l'acide carboxylique comme le composant A, chacun contenu dans la composition de maquillage, est 0,21 ou plus.

4. Composition de maquillage huile dans l'eau selon l'une quelconque des revendications 1 à 3, dans laquelle le composant (A) est un acide carboxylique ayant un groupe hydrocarbure saturé linéaire ou à chaîne ramifiée.

5. Composition de maquillage huile dans l'eau selon la revendication 4, dans laquelle le composant (A) a un groupe carboxyle à une extrémité terminale d'une chaîne polyéthylène.

6. Formulation de mascara comprenant la composition de maquillage huile dans l'eau selon l'une quelconque des revendications 1 à 5.

7. Utilisation de la composition huile dans l'eau selon l'une quelconque des revendications 1 à 5 comme une composition de maquillage.
